(19) Europäisches Patentamt
European Patent Office
Office européen des brevets

(11) **EP 1 967 172 B1**

(12) **EUROPEAN PATENT SPECIFICATION**

(45) Date of publication and mention
of the grant of the patent:
**04.11.2015 Bulletin 2015/45**

(51) Int Cl.:
*A61K 8/02* *(2006.01)*      *A61K 8/73* *(2006.01)*
*A61Q 1/00* *(2006.01)*      *A61Q 1/12* *(2006.01)*

(21) Application number: **08250744.3**

(22) Date of filing: **05.03.2008**

(54) **Cosmetic ingredient and cosmetic composition containing the same**

Kosmetischer Inhaltsstoff und diesen enthaltende kosmetische Zusammensetzung

Ingrédient cosmétique et composition cosmétique l'utilisant

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **09.03.2007 JP 2007060845**

(43) Date of publication of application:
**10.09.2008 Bulletin 2008/37**

(73) Proprietor: **JNC Corporation
Chiyoda-ku
Tokyo (JP)**

(72) Inventors:
• **Yoshida, Naoyuki
Ichihara-shi,
Chiba 290-8551 (JP)**

• **Ishida, Kazushi
Ichihara-shi,
Chiba 290-8551 (JP)**
• **Yoda, Akiko
Ichihara-shi,
Chiba 290-8551 (JP)**

(74) Representative: **Thurston, Joanna et al
Withers & Rogers LLP
4 More London Riverside
London SE1 2AU (GB)**

(56) References cited:
**JP-A- 61 189 210      JP-A- 2006 274 245**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The invention relates to a cosmetic ingredient containing spherical sulfated cellulose or a salt thereof, and a cosmetic composition containing the same.

Background Art

Hyaluronic acid is a polysaccharide existing in dermal connective tissue, which has humectant properties and is able to keep skin elastic, moist and fresh. It is known, however, that hyaluronic acid is decomposed by a hyaluronidase enzyme. In case of shortage of hyaluronic acid in skin due to degradation of hyaluronic acid, the skin becomes dry causing rough skin. "Sensitive skin", which has been increasing recent years, describes skin conditions excessively sensitive to chemical ingredients in cosmetics. This is initiated by drying of skin, which destroys cell surfaces and extracellular matrix to expose the cells, and such skin is further damaged by a pathogen, an inflammatory mediator, an anti-inflammatory drug, an antiseptic agent and other various substances.

While sulfated polysaccharides are known as compounds to inhibit the activity of hyaluronidase (e.g. Japanese patent Laid-Open No. 2000-178196). It is considered that sulfated polysaccharides can promote regeneration of cell surfaces and matrix of protective, connective tissue by inhibiting the activity of hyaluronidase, and, as a result, provide antiinflammation or tissue regeneration functions. As a representative example of such sulfated polysaccharides, chondroitin sulfate is known. The inventors discovered that sulfated cellulose has hyaluronidase inhibitory potency 100 times as strong as chondroitin sulfate (Japanese Patent Laid-Open No. 2006-274245). However, further investigation has been required for better spreadability and smoother touch for the use as a cosmetic ingredient.

According to practice in toileting, makeup cosmetics, such as a foundation, are left put on skin nearly all day long, and therefore the great strain works on skin. On the other hand, such makeup cosmetics as foundations can function as a trouble-free and easy means, by which protective and restorative effects are automatically expected. Due to recent expansion of a sphere of social activities of users, accompanied by their versatile life-styles, said protective and restorative functions of the makeup cosmetics have been attracting more attention.

Disclosure of the Invention

Under such circumstances, it has been desired that a cosmetic ingredient be provided, which has high hyaluronidase inhibitory potency, is capable of presenting effective protective and restorative functions to skin, and can be formulated in various product forms of cosmetics.

The inventors studied intensively for achieving the object to discover that spherical sulfated cellulose, produced by sulfate esterification of a part of hydroxy groups of spherical cellulose, has hyaluronidase inhibitory potency and good spreadability as well as smooth touch, and can be suitably formulated in various product forms of cosmetics, thereby completing the invention.

Namely the invention provides a cosmetic ingredient and cosmetic composition as described below.

[1] A cosmetic ingredient including spherical sulfated cellulose or a salt thereof.

[2] The cosmetic ingredient according to item [1], which has hyaluronidase inhibitory potency.

[3] The cosmetic ingredient according to item [1] or [2] above, wherein the average particle diameter of the spherical sulfated cellulose or the salt thereof is approximately 0.01 to approximately 45 $\mu$m.

[4] The cosmetic ingredient according to any one of items [1] to [3], wherein the sphericity of the spherical sulfated cellulose or the salt thereof is approximately 0.8 to approximately 1.0.

[5] The cosmetic ingredient according to any one of items [1] to [4], wherein the sulfur content of the spherical sulfated cellulose or the salt thereof is approximately 0.001 to approximately 10 weight %.

[6] The cosmetic ingredient according to any one of items [1] to [5], wherein the salt of the spherical sulfated cellulose is one or more selected from the group consisting of a lithium salt, a potassium salt, a sodium salt, a beryllium salt, a magnesium salt, a calcium salt, a triethylamine salt, an arginine salt, a lysine salt and a histidine salt.

[7] A cosmetic composition including the cosmetic ingredient according to any one of items [1] to [6].

[8] The cosmetic composition according to item [7], wherein the content of the spherical sulfated cellulose or the salt thereof is approximately 0.01 to approximately 50 weight %.

According to the invention, a cosmetic ingredient, which has hyaluronidase inhibitory potency and good spreadability and smooth touch, can be obtained. The cosmetic ingredient according to the invention can be suitably used in various product forms of cosmetics. For example, the cosmetic ingredient according to the invention can be suitably formulated in skin care cosmetics, makeup cosmetics and skin cleansing agents.

Brief Description of the Drawing

Figure 1 shows a graph comparing the inhibition ratios of the activity of hyaluronidase at various concentrations of spherical sulfated cellulose sodium salts prepared in Examples 1 and 2 hereof suspended in deionized water, and the inhibition ratios of the activity of hyaluronidase at various concentrations of spherical cellulose prepared in Comparative Example 1 hereof suspended in deionized water.

Best Mode for Carrying Out the Invention

The cosmetic ingredient and the cosmetic composition of the invention will be described in more detail.

A. The cosmetic ingredient

The cosmetic ingredient of the invention includes spherical sulfated cellulose or a salt thereof. The cosmetic ingredient of the invention includes spherical sulfated cellulose or a salt thereof, which has hyaluronidase inhibitory potency, provides excellent humectant properties, good spreadability and smooth touch, and can be applied to skin without imposing strain on the skin such that the skin can be effectively protected against/recovered from dry and rough skin. Further, the cosmetic ingredient of the invention can be easily dispersed in cosmetics, and it is suitable for use not only in skin care cosmetics, but also in makeup cosmetics, skin cleansing agents and other various product forms of cosmetics.

The spherical sulfated cellulose or its salt to be used under the invention is a compound derived by sulfate esterification of at least a part of hydroxy groups of spherical cellulose. Although there are no restrictions on salts of spherical sulfated cellulose under the invention insofar as the object of the invention is not impaired, it is preferable to select one or more out of a lithium salt, a potassium salt, a sodium salt, a beryllium salt, a magnesium salt, a calcium salt, a triethylamine salt, an arginine salt, a lysine salt and a histidine salt. Especially preferable is a sodium salt.

Owing to the spherical form of the spherical sulfated cellulose or its salt to be used under the invention, the cosmetics containing the same are endowed with good spreadability and smooth touch, and owing to the large surface area the hyaluronidase inhibitory potency can be more efficiently exhibited. In this Specification the term "spherical" includes forms from perfect sphere to approximate sphere. The sphericity of the spherical sulfated cellulose or its salt to be used under the invention is preferably approximately 0.8 to approximately 1.0, and more preferably approximately 0.9 to approximately 1.0. The spherical sulfated cellulose or its salt having the above sphericity range endows the host cosmetics with good spreadability and smooth touch. In this Specification the term "sphericity" refers to a ratio of minor axis/major axis of the spherical sulfated cellulose or its salt. The sphericity can be determined by measuring the minor axis/major axis of dried particles under an optical microscope.

There are no restrictions on the average particle size of the spherical sulfated cellulose or its salt to be used under the invention, which may be appropriately decided in accordance with a product form or use. For example, for the use in makeup cosmetics, the average particle size is preferably approximately 0.01 to approximately 45 $\mu$m, more preferably approximately 1 to approximately 45 $\mu$m, further preferably approximately 1 to approximately 20 $\mu$m. The above range of the average particle size gives such advantages that spreadability and smooth touch can be further improved and the hyaluronidase inhibitory potency can be more effectively exhibited. Further adhesion to skin is good and transparency is superior, which are favorable for use in makeup cosmetics. In the use in makeup cosmetics, to avoid rough skin feeling when applied on skin, it is preferable that the particle size of the spherical sulfated cellulose or its salt is uniform and the maximum particle size is below approximately 50 $\mu$m.

For the use in skin care cosmetics and skin cleansing agents, the average particle size is preferably approximately 0.01 to approximately 350 $\mu$m, more preferably approximately 1 to approximately 300 $\mu$m. The above range of the average particle size provides smooth touch and desired humectant properties. It is also preferable in the use in skin care cosmetics and skin cleansing agents that the particle size of the spherical sulfated cellulose or its salt is uniform and the maximum particle size is below approximately 400 $\mu$m.

The average particle size of the spherical sulfated cellulose or its salt can be measured by a Laser diffraction/scattering particle size distribution analyzer.

The sulfur content of the spherical sulfated cellulose or its salt to be used under the invention is preferably approximately 0.001 to approximately 10 weight % based on the weight of the spherical sulfated cellulose or its salt, more preferably approximately 0.01 to approximately 1 weight %, further preferably approximately 0.1 to approximately 0.3 weight %. With sulfur content higher than approximately 0.5 weight % it may become difficult to maintain a spherical form, however, by cross-linking the raw material spherical cellulose, it is possible to increase the sulfur content, while maintaining the spherical form.

The spherical sulfated cellulose or its salt to be used under the invention can be obtained by treating spherical cellulose in a solvent with a sulfation agent for sulfate esterification.

There are no restrictions on spherical cellulose used as a raw material for the spherical sulfated cellulose or its salt to be used under the invention, and generally known crosslinked or non-crosslinked spherical cellulose can be employed. The spherical cellulose is solid particles of spherical forms, which preferable sphericity is approximately 0.8 to approximately 1.0, more preferable is approximately 0.9 to approximately 1.0. The sphericity of the spherical cellulose is measured by a method similar to the afore-mentioned method for the sphericity of the spherical sulfated cellulose.

The spherical cellulose can be produced by dissolving cellulose and regenerating the same. For example, it can be produced by a method via an acetate ester as disclosed by Japanese Patent Publication No. Sho 55-39565 or Japanese Patent Publication No. Sho 55-40618, by a method of pelletizing from a solution with calcium thiocyanate as disclosed in Japanese Patent Publication No. Sho 63-62252, a method of producing from a solution of paraformaldehyde/dimethylsulfoxide as disclosed in Japanese Patent Publication No. Sho 59-38203, or a method of forming cellulose from a solution of cellulose dissolved in an amide containing lithium chloride as disclosed in Japanese Patent No. 3663666.

The spherical cellulose under the invention may be crosslinked or non-crosslinked. The crosslinked spherical cellulose can be obtained by treating non-crosslinked spherical cellulose by a crosslinking agent according to a conventional method. As a crosslinking agent, a multifunctional epoxy compound, such as epichlorohydrin, can be used.

There are no restrictions on the average particle size of the spherical cellulose to be used under the invention, however to produce the spherical sulfated cellulose with a desired average particle size, it is preferably approximately 45 $\mu$m or less, more preferably approximately 1 to approximately 45 $\mu$m, further preferably approximately 1 to approx- imately 20 $\mu$m.

Under the invention a commercially available spherical cellulose can be used. For example "Celluflow C-25" (Trade name of Chisso Corp., average particle size 8 to 12 $\mu$m) and "Celluflow TA-25" (Trade name of Chisso Corp., average particle size 4 to 12 $\mu$m) based on the raw materials of non-crosslinked cellulose can be used.

The spherical cellulose to be used under the invention contains usually about 2 to 3 weight % of water based on the total own weight. Sulfate esterification reaction of such wet spherical cellulose may not proceed effectively due to deactivation of a sulfate esterification agent by the moisture. Therefore it is preferable to remove the moisture as much as possible by drying the spherical cellulose prior to the sulfate esterification reaction.

There are no restrictions on the method for removing the moisture, insofar as the moisture in the spherical cellulose can be sufficiently removed without destroying the form of the spherical cellulose. For example, heat drying can be employed for moisture removal. Such heat drying may be carried out under reduced pressure.

Heat drying should preferably be continued until the water content in the spherical cellulose is approximately 1 weight % or less, which enables an efficient sulfate esterification reaction. The heating temperature is preferably approximately 80 $\pm$ 20°C.

As a sulfation agent to be used for sulfate esterification of the spherical cellulose under the invention, for example, sulfuric anhydride, or a mixture of N,N-dimethylformamide and sulfuric anhydride (including a complex formed by N,N-dimethylformamide and sulfuric anhydride) is preferable. If a mixture of N,N-dimethylformamide and sulfuric anhydride is used, the concentration of the sulfuric anhydride in the mixture is preferably approximately 1 to approximately 30 weight %, more preferably approximately 5 to approximately 20 weight %, and further preferably approximately 18 weight %.

The amount of the sulfation agent to be used can be appropriately decided within the range required for sulfate esterification of the spherical cellulose, which range can be easily decided by those skilled in the art. For example, the mass of the spherical cellulose is divided by molecular weight of glucose, which is deemed as a structural unit, and the quotient is assumed as a mol number of the spherical cellulose. The amount of sulfuric anhydride in the sulfation agent is preferably adjusted to the amount determined by multiplying said mol number by approximately 0.01 to approximately 5 mol, or more preferably the amount of sulfuric anhydride in the sulfation agent should be adjusted to the amount determined by multiplying the same by approximately 0.1 to approximately 1 mol. The amount of introduction of sulfuric groups (sulfur concentration) into spherical sulfated cellulose or its salt to be used under the invention can be controlled by appropriately adjusting the feeding amount of the sulfation agent in relation to the spherical cellulose.

There are no restrictions on a solvent to be used for sulfate esterification reaction of spherical cellulose, insofar as a solvent is inert to the spherical cellulose or a sulfation agent, and heterocyclic solvents, such as N,N-dimethylformamide, dimethyl sulfoxide, dioxane and pyridine, and tertiary amine solvents, such as triethylamine, are preferably used. The amount of the solvent to be used may be decided appropriately in accordance with reaction conditions, and is preferably approximately 1 to approximately 100 times as much as the weight of the spherical cellulose, more preferably approximately 1 to approximately 10 times as much.

The sulfate esterification reaction is carried out by dropping the sulfation agent to a spherical cellulose suspension in the solvent, which has been prepared beforehand. The reaction is preferably carried out with agitation to conduct the reaction evenly. The reaction temperature need not be low, and is preferably in the range of approximately 0 to approximately 70°C, more preferably in the range of approximately 0 to approximately 50°C, further preferably in the range of approximately 30 to approximately 50°C. There are no restrictions on a reaction time, insofar as the sulfate esterification reaction sufficiently proceeds. Standard reaction time is approximately 1 to approximately 10 hours, preferably approximately 2 to approximately 6 hours, and more preferably approximately 2 to approximately 4 hours.

After completion of the reaction, the reactant product is recovered by filtration etc. and washed with an alcohol solvent, such as methanol or ethanol, to obtain spherical sulfated cellulose.

The obtained spherical sulfated cellulose may be used as it is, but preferably it should be neutralized with alkali, such as an inorganic base, an organic base and a basic amino acid. By neutralization salts of spherical sulfated ester are formed, thereby decomposition of spherical sulfated cellulose by a sulfate ester group can be suppressed.

Examples of salts of spherical sulfated cellulose formed by neutralization with inorganic bases are: a lithium salt, a potassium salt, a sodium salt, a beryllium salt, a magnesium salt and a calcium salt. An example of salts of spherical sulfated cellulose formed by neutralization with organic bases is: a triethylamine salt. Examples of salts of spherical sulfated cellulose formed by neutralization with basic amino acids are: an arginine salt, a lysine salt and a histidine salt. Among them a sodium salt is favorably used, since its production is easy and the price is rather reasonable.

After neutralization, the reactant product is washed with deionized water to obtain the target spherical sulfated cellulose salt. The spherical sulfated cellulose salts under the invention include both anhydrides and hydrates.

The spherical sulfated cellulose or its salt to be used under the invention is in the form of dry powders, but due to its high water-holding capacity, it may contain approximately 2 to approximately 3 weight % moisture based on its total weight, since complete removal of the water is impossible, or after removal it may reabsorb moisture from the atmosphere. Under the invention such moist spherical sulfated cellulose or salt can also be used suitably.

The cosmetic ingredient of the invention may use a single type of spherical sulfated cellulose or its salt, or in combination of 2 or more types.

B. Cosmetic Composition

Next, the cosmetic composition of the invention will be described.

The cosmetic composition of the invention contains the cosmetic ingredient described in "A. Cosmetic Ingredient" hereof, and consequently provides excellent humectant properties, while maintaining good spreadability and smooth touch.

There are no restrictions on the content of the cosmetic ingredient in the cosmetic composition of the invention, which should be decided in consideration of the balance among the concentration to be selected for the required humectant properties, the relevant raw material costs, and further an object of the product. The content of the cosmetic ingredient, as the content of the spherical sulfated cellulose or its salt with respect to the total weight of the cosmetic composition of the invention, is discretionarily decided preferably in the range of approximately 0.0001 to approximately 99 weight %, more preferably approximately 0.001 to approximately 80 weight %, further preferably approximately 0.001 to approximately 70 weight %, and especially preferably approximately 0.01 to approximately 50 weight %. For example, in case of a liquid foundation the content is preferably approximately 0.001 to approximately 10 weight %, and in case of a solid foundation it is preferably approximately 0.001 to approximately 50 weight %.

The cosmetic composition of the invention may contain in addition to the cosmetic ingredient of the invention other additives as may be required. There are no restrictions on the additives to be added to the cosmetic composition of the invention insofar as the object of the invention is not deviated, and suitable standard cosmetic ingredients can be added.

Examples of effective ingredients to be added are: pigments, pigmentation inhibitors, tyrosinase inhibitors, melanocyte melanin production inhibitors, melanin production promoters, humectants other than spherical sulfated cellulose and its salts, cell- and metabolism activators, antioxidants, active oxygen scavengers/radical formation retarders, lipid metabolism accelerators, UV filters/UV absorbers, astringents, anti-inflammatory agents/interleukin production inhibitors/antiphlogistic agents, antiseborrheic agents, antibacterial agents/antiviral agents, blood flow improvers/vascular stimulants, antiandrogenic agents, inhibitors of structural protein proteases (elastase, collagenase, keratin protease, serine protease, integrin degrading enzyme, involucrin degrading enzyme, fillagrin degrading enzyme, laminin degrading enzyme, fibronectin degrading enzyme, proteoglycan degrading enzyme, etc.), structural protein synthesis accelerating agents, degrading enzyme inhibitors of mucopolysaccharides (hyaluronic acid, chondroitin sulfate, etc.), mucopolysaccharide synthesis accelerating agents, intercellular lipid generation accelerating agents/intercellular lipid condition improvers, keratolytic agents/homy layer exfoliation agents, plasminogen activator antagonists - inhibitors, Maillard reaction inhibitors, testosterone $5\alpha$-reductase inhibitors/dermal papilla activators/hair regrowth agents, hair-matrix cell growth inhibitors/hair growth inhibitors, hair swelling agents/hair protection agents, and deodorants.

Other substances favored as ingredients for cosmetic composition, such as extracts, metabolites derived from plant-based raw materials, animal-based raw materials, microbe-based raw materials, or the other natural raw materials, and other compounds may be discretionarily selected as additives for use in combination. As an example for the aforementioned additives a compound is disclosed in Japanese Patent Laid-Open No. 2005-350454.

There are no restrictions on the content of the additives, which may be decided appropriately in accordance with the use and object, but typically a preferable content is in the range of approximately 0.0001 to approximately 50 weight % based on the total weight of the cosmetic composition of the invention, more preferably approximately 0.001 to approximately 50 weight %, and further preferably approximately 0.01 to approximately 50 weight %.

The cosmetic composition of the invention may adopt any form, such as fine powder, fine crystallite, liquid and pellet. Such form is selected appropriately according to the product form. For example, using optional additives, such as an excipient, a thickener and a gelling agent, granular, jelly, or viscous fluid preparations are possible. For an excipient, a thickener and a gelling agent, any of such compounds as are generally used as additives to cosmetic compositions, may be appropriately selected in accordance with an object of the use.

The cosmetic composition of the invention can be produced by measuring and mixing the cosmetic ingredient of the invention and other necessary additives to the portions to be decided in accordance with the use. The production apparatus and the production conditions may be selected according to the properties and use of the target cosmetic composition out of generally known production apparatuses and production conditions.

The cosmetic composition of the invention can be used for various product forms, and are, among others, suitable for skin care cosmetics, makeup cosmetics and skin cleansing agents. Since the cosmetic composition of the invention includes the cosmetic ingredient of the invention with hyaluronidase inhibitory potency, when formulated to skin care cosmetics, it provides excellent skin protection/recovery properties. Further, the cosmetic ingredient as a humectant provides good spreadability and smooth touch, it can be suitably formulated to makeup cosmetics, such as a foundation or a white makeup powder, to provide an excellent feeling at application. Although makeup cosmetics is put on skin nearly all day long, and therefore great strain is imposed on skin, the use of the cosmetic composition of the invention, containing the cosmetic ingredient of the invention having high hyaluronidase inhibitory potency providing excellent skin protection/recovery properties, can litigate such strain on skin. Further, since the cosmetic composition of the invention can be applied on skin without imposing strain on skin, it can be suitably formulated to a skin cleansing agent with minimized damages on skin during skin cleansing.

Favorable examples of skin care cosmetics are: a cosmetic water, a serum, a whitening cosmetic water, a milky lotion, a whitening milky lotion, a cream, a whitening cream, a salve, a whitening salve, a lotion, a whitening lotion, a cosmetic oil and a cosmetic pack.

Favorable examples of makeup cosmetics are: a solid foundation, a liquid foundation, a lipstick, a lip gloss, an eye-shadow, a white makeup powder, a blusher, an eyeliner, a mascara and an eyebrow color.

Favorable examples of skin cleansing agents are: a soap, a cleansing cream, a cleansing lotion, a cleansing milk, a facial cleanser and a body shampoo.

There are no restrictions on the constitutions of the cosmetics insofar as the cosmetic ingredient according to the invention is included, and the publicly known constitutions can be used for the respective cosmetics. For examples the following sources can be used as references: "Koushouhin Kagaku (Cosmetic Chemistry) - Theory and Practice- (4th-edition)" (Hiroshi Hirota, Yasuo Tamura; Fragrance Journal Ltd.), "Cosmetics Handbook" (Shigeru Sekine et al.; Nikko Chemicals Co. Ltd. et al.), Japanese Patent Laid-Open No. 2005-200407, Japanese Patent Laid-Open No. Hei 1-143816, Japanese Patent Laid-Open No. 2000-191442, Japanese Patent Laid-Open No. 2005-314393, Japanese Patent Laid-Open No. 2000-319629, Japanese Patent Laid-Open No. 2003-160465, Japanese Patent Laid-Open No. 2005-232049.

Examples

The invention will now be described in more detail by way of Examples and Comparative Example. The following examples are for illustrative purposes only and are not intended, nor should they be interpreted to, limit the scope of the invention.

[Example 1]

Production of spherical sulfated cellulose sodium salt

As a starting material, spherical cellulose tradenamed as Celluflow C-25 (Chisso Corp., average particle size 8 to 12 μm) was used. Celluflow C-25 was firstly dried by a vacuum drier at 80°C, so that the water content in the spherical cellulose was lowered below 1 weight %. The final water content was 0.7 weight %. The dried powder was used for sulfate esterification.

To 150 mL of N,N-dimethylformamide 48.99 g of the dried powder of spherical cellulose was charged, and into the mixture liquid 61.00 g of an 18 weight % solution of sulfuric anhydride in dimethylformamide solvent was gradually dropped at room temperature. The mixture was stirred at a reaction temperature of 32°C for 4 hours after completion of the dropping. The used 18 weight % solution of sulfuric anhydride in dimethylformamide solvent was prepared beforehand by dropping 278 g of sulfuric anhydride with agitation into 2500 g of N,N-dimethylformamide cooled to 5°C or less in an ice bath.

After the completion of the reaction, the reacted solution was filtered and the filtrand was washed by methanol, charged into deionized water cooled to 10°C or less, and neutralized with 1M NaOH. The solid was washed thoroughly with deionized water and spherical sulfated cellulose sodium salt was obtained.

According to elementary analysis the sulfur content measured was 0.18 weight %. The measured average particle size of the product spherical sulfated cellulose sodium salt was 11.3 $\mu$m, and the sphericity was 0.9.

The product spherical sulfated cellulose sodium salt was suspended in deionized water to the concentrations of 0.1 weight %, 0.5 weight %, 1.0 weight % respectively, and used in the following test of hyaluronidase inhibition.

[Example 2]

Production of spherical sulfated cellulose sodium salt

As a starting material, spherical cellulose with the average particle size of 250 $\mu$m was used. Its production steps are as follows:

(1) Into an aqueous solution of 60 weight % (as anhydride) of calcium thiocyanate, 0.46 kg of crystalline cellulose is added and dissolved by heating up to 110°C.
(2) The solution, after added with a surfactant, is dropped into 30 L of o-dichlorobenzene preheated to 130 to 140°C, and then the solution is agitated to be dispersed.
(3) The dispersion solution is cooled below 40°C, to which 13 L of methanol is added to precipitate particles.
(4) The suspension is filtered, and the particles are washed with 13 L of methanol and then filtered again. The procedures are repeated several times.
(5) After washing with a large amount of water, the object product of spherical cellulose powders are obtained.

In order to remove the water in the spherical cellulose, 1942.4 g (wet base) was weighed and placed into a 5000 mL beaker, to which 4000 mL of N,N-dimethylformamide was added. The mixture was stirred for 30 minutes, settled, and then the water content in the supernatant was measured by the Karl Fisher method. The water removal procedure was repeated until the water content in the supernatant was lowered in the range of 0.2 weight %. The final water content in the supernatant was 0.19 weight %, and the dried spherical cellulose was used in the following sulfate esterification step.

The dried spherical cellulose was dispersed in N,N-dimethylfonnamide cooled to 5°C or less in an ice bath, to which 370.0 g of 18 weight % solution of sulfuric anhydride in dimethylformamide precooled to 5°C was gradually dropped. The mixture was stirred for 4 hours maintaining the reaction temperature of 30$\pm$2°C. After the completion of the reaction, the reacted solution was filtered and the filtrand was washed by methanol. The used 18 weight % solution of sulfuric anhydride in dimethylformamide was prepared according to the method described in Example 1.

The filtrand was then neutralized with 1M NaOH in deionized water cooled to 10°C or less. Thereafter the solid was washed with deionized water and spherical sulfated cellulose sodium salt was obtained.

According to elementary analysis the sulfur content measured was 2.8 weight %. The measured average particle size of the product spherical sulfated cellulose sodium salt was 250 $\mu$m, and the sphericity was 0.9.

The product spherical sulfated cellulose sodium salt was suspended in deionized water to the concentrations of 0.1 weight %, 0.5 weight %, 1.0 weight % respectively, and used in the following test of hyaluronidase inhibition.

[Comparative Example 1]

As a comparative example, instead of a spherical sulfated cellulose a non-sulfated spherical cellulose tradenamed as Celluflow C-25 (Chisso Corp., average particle size 8 to 12 $\mu$m) was used, which was suspended in deionized water to the concentrations of 0.1 weight %, 0.5 weight %, 1.0 weight % respectively.

[Test Example 1]

Tests of inhibition of bovine testis-derived hyaluronidase activity

Using the suspensions of various concentrations prepared according to Examples 1 and 2, and Comparative Example 1, tests of inhibition of bovine testis-derived hyaluronidase activity were carried out. The reagents used in the tests were manufactured by Wako Pure Chemical Industries, Ltd., unless otherwise specified.

The following 6 Solutions A to F were prepared for use in the tests.

Solution A: A solution (concentration 2.83 mg/mL) of hyaluronidase originated from bovine testis (Sigma-Aldrich Co.) in 0.1 mol/L acetate buffer solution (pH 4.0),

Solution B: 0.3 mol/L sodium chloride - 0.1 mol/L acetate buffer solution (pH 4.0),

Solution C: A solution (concentration 1.83 mg/mL) of sodium hyaluronate (Chisso Corp., CHA H-Type) in 0.1 mol/L acetate buffer solution (pH 4.0),

Solution D: A 0.4 mol/L aqueous solution of sodium hydroxide,

Solution E: A 0.8 mol/L aqueous solution of sodium borate, and

Solution F: A solution of 1 g of p-dimethylaminobenzaldehyde in 1.25 mL of 10 N hydrochloric acid and 98.75 mL of acetic acid.

(Preparation of Test Solutions)

Test solutions were prepared with the suspensions of various concentrations prepared according to Examples 1 and 2, and Comparative Example 1.

Firstly, 0.2 mL of Solution B was added to 0.025 mL of Solution A, and the mixture was kept at 37°C for 20 minutes. The above suspension was added to the mixture, which was then left standing in a thermostat at 37°C for 20 minutes. Then, 0.2 mL of Solution C was added to the mixture, which was then left standing in a thermostat at 37°C for 20 minutes. After adding 0.1 mL of Solution D and 0.1 mL of Solution E, the mixture was boiled for 3 minutes and cooled down, and 3.0 mL of Solution F was added. The mixture was left standing in a thermostat at 37°C for 20 minutes to complete a test solution. The absorbance $Q_E$ at 585 nm of the test solution was measured using reductive terminal N-acetylhexosamine generated by degradation of the hyaluronidase as an index, and pure water as a reference.

(Preparation of Control Solution 1)

Control Solution 1 was prepared identically with the test solution, except that 0.1 mol/L acetate buffer solution (pH 4.0) instead of Solution A, and pure water instead of a spherical sulfated cellulose sodium salt were used. The absorbance $Q_1$ at 585 nm of Control Solution 1 was measured as in the case of the test solution.

(Preparation of Control Solution 2)

Control Solution 2 was prepared identically with the test solution, except that pure water instead of a spherical sulfated cellulose sodium salt suspension was used. The absorbance $Q_2$ at 585 nm of Control Solution 2 was measured as in the case of the test solution.

The inhibition ratios of the activity of hyaluronidase were calculated according to the following equation for the measured absorbance values of $Q_E$, $Q_1$ and $Q_2$, with respect to the suspensions of spherical sulfated cellulose sodium salt in deionized water with the concentrations of 0.1 weight %, 0.5 weight % and 1.0 weight % prepared in Examples 1 and 2, and the suspensions of spherical cellulose "Celluflow C-25" in deionized water with the concentrations of 0.1 weight %, 0.5 weight % and 1.0 weight % prepared in Comparative Example 1.

$$\text{Inhibition ratio (\%)} = \{(Q_2 - Q_1) - (Q_E - Q_1)\}/(Q_2 - Q_1)$$

The results are shown in Figure 1.

As obvious from the results shown in Figure 1, Example 1 and Example 2 using spherical sulfated cellulose showed hyaluronidase inhibitory potency, but Comparative Example 1 using spherical cellulose did not show hyaluronidase inhibitory potency.

The inhibition ratio of the activity of hyaluronidase increased in proportion to the concentration of spherical sulfated cellulose sodium salt. In Example 2 at the concentration 0.1 weight % of spherical sulfated cellulose sodium salt, the hyaluronidase inhibitory potency was not shown, however, at the concentration of 0.5 weight % an inhibition ratio of nearly 20% was obtained indicating the high inhibition potency even at a low concentration of 1 weight % or less.

Example 1 showed about 4 times as high hyaluronidase inhibitory potency as Example 2. Comparing the sulfur concentrations in the spherical sulfated cellulose sodium salts, it was 0.18 weight % in Example 1, and 2.8 weight % in Example 2, namely the sulfur concentration in Example 1 was about 1/15 of that in Example 2, while hyaluronidase inhibitory potency of Example 1 was higher.

The results show that high hyaluronidase inhibitory potency was endowed by sulfation of spherical cellulose. At the same time, hyaluronidase inhibitory potency was not enhanced in simple proportion to the sulfur concentration in the spherical sulfated cellulose, but the decrease in the average particle size increased the potency by 4 times.

Presumably, the decrease in the average particle size of the spherical sulfated cellulose increased the surface area, which resulted in higher hyaluronidase inhibitory potency.

As demonstrated above, spherical sulfated cellulose or its salt can provide high hyaluronidase inhibitory potency even at lower concentration. Such effect can be strengthened by reducing the average particle size. By mixing the spherical sulfated cellulose or its salt in cosmetics, a function of inhibiting degradation of hyaluronic acid is added, and the cosmetics can have skin humectant properties together with antiinflammatory properties or tissue-regeneration properties. Further, since the spherical sulfated cellulose or its salt provides good spreadability and smooth touch, it can be formulated suitably in cosmetics.

Product examples of the cosmetic composition of the invention and the sensory test results will be described below:

| (Product Example 1) Liquid foundation | (weight %) |
|---|---|
| decamethylcyclopenta-siloxane | 16.0 |
| dimethylpolysiloxane | 8.0 |
| 12-hydroxy stearic acid | 1.0 |
| fluorinated silicone | 5.0 |
| spherical silicone resin powder | 3.0 |
| fluorine-compound treated micro-titanium dioxide | 8.0 |
| fluorine-compound treated titanated mica | 1.0 |
| fluorine-compound treated titanium dioxide | 5.0 |
| fluorine-compound treated iron (III) oxide | 0.9 |
| fluorine-compound treated iron (III) oxide-hydroxide | 2.0 |
| fluorine-compound treated iron (II, III) oxide | 1.0 |
| ethanol | 15.0 |
| the compound obtained in Example 1 | 0.5 |
| 1,6-hexanediol | 2.0 |
| glycerin | 3.0 |
| magnesium sulfate | 1.0 |
| flavor | 0.05 |
| purified water | balance |

| (Product Example 2) Liquid foundation | (weight %) |
|---|---|
| decamethylcyclopenta-siloxane | 16.0 |
| dimethylpolysiloxane | 8.0 |
| 1,3-butanediol | 5.0 |
| PEG-7 dimeticone | 1.0 |
| the compound obtained in Example 1 | 1.0 |
| glycerin | 1.0 |
| cyclohexasiloxane | 2.0 |
| cyclopentasiloxane | 0.5 |
| dimeticone | 1.0 |
| stearic acid | 1.0 |
| talc | 2.0 |
| tocopherol | 0.1 |
| dextrin palmitate | 2.0 |
| sodium hyaluronate | 0.1 |
| phenoxyethanol | 0.1 |
| poly(methyl methacrylate) | 5.0 |
| mica | 5.0 |
| titanium dioxide | 8.0 |
| iron oxide | 1.0 |

(continued)

| (Product Example 2) Liquid foundation | (weight %) |
|---|---|
| magnesium sulfate | 1.0 |
| purified water | balance |

| (Product Example 3) White makeup powder | (weight) |
|---|---|
| the compound obtained in Example 1 | 15.0 g |
| kaolin | 1.0 g |
| micro titanium dioxide | 0.6 g |
| zinc myristate | 1.0 g |
| magnesiun carbonate | 1.0 g |
| sericite | 1.4 g |
| 20% iron (III) oxide in talc | 0.2 g |
| 20% iron (III) oxide-hydroxide in talc | 0.2 g |

[Sensory Test 1]

The liquid foundation of Product Example 1 was used by 14 females in the age range between 30 and 69 for 3 days, and their evaluation results were expressed with the following 5 grade scores. The average scores are shown in Table 1.

- excellent    5
- fair    4
- ordinary    3
- poor    2
- bad    1

Table 1: Result of sensory test

| | Evaluation item | Average score |
|---|---|---|
| 1 | Did it spread well over skin? | 4.1 |
| 2 | When applied, was skin felt moist? | 3.7 |
| 3 | Were pores (irregularities in the skin) covered well? | 3.6 |
| 4 | Did moisturization last? | 3.6 |
| 5 | Do you like the foundation overall? | 3.6 |

Table 1 shows that the cosmetic composition of the invention has been valued highly in the items of spreadability, moisturizing properties and persistence, and covering properties, and that it is suitable for cosmetics.

Industrial Applicability

The cosmetic ingredient of the invention provides excellent humectant properties and good spreadability and smooth touch. The cosmetic ingredient of the invention is suitable for use, not only in skin care cosmetics, but also in makeup cosmetics, skin cleansing agents and other various product forms of cosmetic compositions.

Although the invention has been described and illustrated with a certain degree of particularity, it is understood that the disclosure has been made only by way of example, and that numerous changes in the conditions and order of steps can be resorted to by those skilled in the art without departing from the spirit and scope of the invention.

**Claims**

1. A cosmetic ingredient comprising spherical sulfated cellulose or a salt thereof.

2. The cosmetic ingredient according to claim 1, which has hyaluronidase inhibitory potency.

3. The cosmetic ingredient according to claim 1 or 2, wherein the average particle diameter of the spherical sulfated cellulose or the salt thereof is 0.01 to 45 $\mu$m.

4. The cosmetic ingredient according to any one of claims 1 to 3, wherein the sphericity of the spherical sulfated cellulose or the salt thereof is 0.8 to 1.0.

5. The cosmetic ingredient according to any one of claims 1 to 4, wherein the sulfur content of the spherical sulfated cellulose or the salt thereof is 0.001 to 10 weight %.

6. The cosmetic ingredient according to any one of claims 1 to 5, wherein the salt of the spherical sulfated cellulose is one or more selected from the group consisting of a lithium salt, a potassium salt, a sodium salt, a beryllium salt, a magnesium salt, a calcium salt, a triethylamine salt, an arginine salt, a lysine salt and a histidine salt.

7. A cosmetic composition comprising the cosmetic ingredient according to any one of claims 1 to 6.

8. The cosmetic composition according to claim 7, wherein the content of the spherical sulfated cellulose or the salt thereof is 0.01 to 50 weight %.

**Patentansprüche**

1. Kosmetischer Bestandteil umfassend sphärische sulfatierte Cellulose oder ein Salz davon.

2. Kosmetischer Bestandteil nach Anspruch 1, welcher Hyaluronidase-hemmende Wirkung aufweist.

3. Kosmetischer Bestandteil nach Anspruch 1 oder 2, wobei der durchschnittliche Teilchendurchmesser der sphärischen sulfatierten Cellulose oder des Salzes davon von 0,01 bis 45 $\mu$m ist.

4. Kosmetischer Bestandteil nach einem der vorhergehenden Ansprüche 1 bis 3, wobei die Sphärizität der sphärischen sulfatierten Cellulose oder des Salzes davon von 0,8 bis 1,0 ist.

5. Kosmetischer Bestandteil nach einem der vorhergehenden Ansprüche 1 bis 4, wobei der Schwefelgehalt der sphärischen sulfatierten Cellulose oder des Salzes davon von 0,001 bis 10 Gew.-% ist.

6. Kosmetischer Bestandteil nach einem der vorhergehenden Ansprüche 1 bis 5, wobei das Salz der sphärischen sulfatierten Cellulose eines oder mehrere ist, ausgewählt aus der Gruppe bestehend aus einem Lithiumsalz, einem Kaliumsalz, einem Natriumsalz, einem Beryllium-Salz, einem Magnesiumsalz, einem Calciumsalz, einem Triethylamin-Salz, einem Arginin-Salz, einem Lysin-Salz und einem Histidin-Salz.

7. Kosmetische Zusammensetzung umfassend einen kosmetischen Bestandteil nach einem der vorhergehenden Ansprüche 1 bis 6.

8. Kosmetische Zusammensetzung nach Anspruch 7, wobei der Gehalt der sphärischen sulfatierten Cellulose oder des Salzes davon von 0,01 bis 50 Gew.-% ist.

**Revendications**

1. Ingrédient cosmétique comprenant de la cellulose sulfatée sphérique ou un sel de ladite.

2. Ingrédient cosmétique selon la revendication 1, qui a un pouvoir inhibiteur de l'hyaluronidase.

**3.** Ingrédient cosmétique selon la revendication 1 ou 2, dans lequel le diamètre moyen de particule de la cellulose sulfatée sphérique ou du sel de ladite est de 0,01 à 45 μm.

**4.** Ingrédient cosmétique selon l'une des revendications 1 à 3, dans lequel la sphéricité de la cellulose sulfatée sphérique ou du sel de ladite est de 0,8 à 1.

**5.** Ingrédient cosmétique selon l'une des revendications 1 à 4, dans lequel la teneur en soufre de la cellulose sulfatée sphérique ou du sel de ladite est de 0,001 à 10% en poids.

**6.** Ingrédient cosmétique selon l'une des revendications 1 à 5, dans lequel le sel de la cellulose sulfatée sphérique est un ou plusieurs sels sélectionnés parmi le groupe consistant en un sel de lithium, un sel de potassium, un sel de sodium, un sel de béryllium, un sel de magnésium, un sel de calcium, un sel de triéthylamine, un sel d'arginine, un sel de lysine et un sel d'histidine.

**7.** Composition cosmétique comprenant l'ingrédient cosmétique selon l'une quelconque des revendications 1 à 6.

**8.** Composition cosmétique selon la revendication 7, dans laquelle la teneur de la cellulose sulfatée sphérique ou du sel de ladite va de 0,01 à 50% en poids.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000178196 A
- JP 2006274245 A
- JP SHO5539565 B
- JP SHO5540618 B
- JP SHO6362252 B
- JP SHO5938203 B
- JP 3663666 B
- JP 2005350454 A

- JP 2005200407 A
- JP HEI1143816 B
- JP 2000191442 A
- JP 2005314393 A
- JP 2000319629 A
- JP 2003160465 A
- JP 2005232049 A

**Non-patent literature cited in the description**

- **HIROSHI HIROTA ; YASUO TAMURA.** Koushouhin Kagaku (Cosmetic Chemistry) - Theory and Practice. Fragrance Journal Ltd,

- **SHIGERU SEKINE et al.** Cosmetics Handbook. Nikko Chemicals Co. Ltd,